# FASCICULE DE BREVET EUROPEEN

(11) **EP 2 792 384 B1**
(45) Date de publication et mention de la délivrance du brevet: **17.06.2015**
(21) Numéro de dépôt: 13185177.6
(22) Date de dépôt: 19.09.2013
(51) Int. Cl.: A61N 1/36

(54) **Dispositif médical implantable actif pour le traitement de l'insuffisance cardiaque avec stimulation du nerf vague**
Aktive implantierbare medizinische Vorrichtung zur Behandlung einer Herzinsuffizienz mit Stimulation des Vagusnervs
Active implantable medical device for treating heart failure with vagus nerve stimulation

(30) Priorité: 19.04.2013 FR 1353582
(43) Date de publication de la demande: 22.10.2014
(73) Titulaire: Sorin CRM SAS, 92140 Clamart Cedex (FR)
(72) Inventeur: Bonnet, Jean-Luc, 91300 Massy (FR)
(74) Mandataire: Dupuis-Latour, Dominique

(56) Documents cités:
- WO-A1-2005/102449
- US-A1- 2010 010 388
- US-A1- 2012 095 530

## Description

L'invention concerne les "dispositifs médicaux implantables actifs" tels que définis par la directive 90/385/CEE du 20 juin 1990 du Conseil des communautés européennes, plus précisément les implants permettant de délivrer des thérapies de stimulation du nerf vague, dites thérapies VNS (*Vagus Nerve Stimulation*).

Elle concerne plus particulièrement l'utilisation de telles thérapies chez des patients en risque d'insuffisance cardiaque.

En effet, la stimulation du nerf vague agit sur les fonctions cardiovasculaires par réduction du rythme cardiaque et de la contractilité du myocarde avec diminution de la durée de la diastole, ce qui peut aider à réduire l'évolution d'un remodelage cardiaque susceptible de mener à une insuffisance cardiaque.

De façon générale, les patients insuffisants cardiaques ont une activité physique réduite, avec un niveau d'effort corrélé à la gravité de la pathologie cardiaque.

Bien que l'activité physique soit reconnue comme étant un moyen thérapeutique pour améliorer la fonction cardiaque, ces patients ont la plupart du temps du mal à produire une activité physique du fait de la réduction de leurs fonctions cardiaques, de sorte qu'ils ne peuvent accomplir que de petits efforts.

À cet égard, du fait de la réduction du rythme cardiaque et de la contractilité du myocarde qu'elle entraine, une thérapie VNS peut être un facteur aggravant de la tolérance à l'effort, car elle réduit la capacité d'adaptation du coeur à l'activité en cours : en effet, en cas d'effort la stimulation VNS empêche le rythme cardiaque de s'accélérer autant que nécessaire, neutralisant ainsi l'effet bénéfique d'une augmentation de l'activité physique.

Pour remédier à cette difficulté il a été proposé, par exemple par le US 2012/0095530 A1, d'adapter la thérapie VNS en fonction du niveau d'activité du patient, ce niveau étant évalué par un capteur approprié tel qu'un accéléromètre intégré au boitier implanté du générateur d'impulsions de stimulation : le signal du capteur est comparé à une série de seuils prédéfinis, et les paramètres de la stimulation VNS, principalement l'énergie de stimulation, sont sélectivement ajustés en fonction des seuils franchis, en particulier pour adapter la thérapie en laissant s'exprimer une accélération du rythme cardiaque corrélée à l'effort.

Toutefois, dans ce dispositif, la modulation de la thérapie VNS est définie en fonction de critères absolus, à savoir les seuils prédéfinis. Or, le niveau d'activité, représenté par un capteur, peut varier de façon significative :
- d'un patient à l'autre, et
- au cours du temps pour un même patient, en fonction de l'évolution à moyen et/ou long terme (par exemple d'un jour à l'autre et/ou sur une période de plusieurs jours ou semaines) de la pathologie dont il est atteint, aussi bien en cas d'aggravation que d'amélioration.

Le but de l'invention est de remédier à cette difficulté, en proposant un dispositif muni d'un asservissement du générateur de stimulation VNS opérant de façon spécifique pour chaque patient grâce à une calibration automatique de cet asservissement, avec en outre possibilité de réitérer cette calibration à intervalles réguliers (par exemple quotidiennement) pour suivre l'évolution de l'état général du patient à moyen et long terme. A cet effet, l'invention propose un dispositif médical pour le traitement de l'insuffisance cardiaque avec stimulation VNS du type général divulgué par le US 2012/0095530 A1 précité, c'est-à-dire comprenant un générateur apte à produire des salves d'impulsions VNS selon plusieurs configurations de thérapie VNS différentes sélectionnables, un capteur apte à générer un signal d'activité fonction du niveau d'activité courante du patient, et des moyens d'asservissement du générateur en fonction du signal d'activité. Ces moyens d'asservissement comprennent un classifieur apte à déterminer dans quelle classe se situe le niveau d'activité courante du patient parmi une pluralité de classes d'activité définies par des bornes respectives du niveau d'activité, et un contrôleur apte à sélectionner une configuration de thérapie VNS respective en fonction de la classe d'activité déterminée par le classifieur.

De façon caractéristique, les bornes définissant les classes d'activité sont des bornes dynamiquement modifiables, et le dispositif comprend des moyens de calibration du classifieur, comportant des moyens d'analyse historique des niveaux d'activité courants successifs du patient pris au cours d'une période d'analyse prédéterminée, et des moyens de définition des bornes des classes d'activité en fonction des résultats de ladite analyse historique.

Le dispositif comprend avantageusement des moyens de mise à jour du classifieur, aptes à activer de manière réitérée, et notamment à intervalles réguliers, les moyens de calibration du classifieur.

Dans une mise en oeuvre préférentielle de l'invention, les moyens de calibration du classifieur comprennent des moyens d'échantillonnage des niveaux d'activité courants successifs du patient au cours de la période d'analyse, des moyens de construction d'un histogramme des niveaux d'activité échantillonnés, et des moyens de partitionnement de l'histogramme en la pluralité de classes d'activité.

De préférence, les moyens d'échantillonnage des niveaux d'activité courants échantillonnent un niveau moyen d'activité au cours de chaque période d'échantillonnage respective.

Les moyens de partitionnement de l'histogramme peuvent notamment comprendre des moyens aptes à définir les bornes entre classes successives de manière que la somme cumulée des niveaux échantillonnés dans chaque classe soit une fraction prédéterminée, de préférence identique pour toutes les classes, de la somme cumulée des niveaux échantillonnés toutes classes confondues.

Dans une forme particulière de mise en oeuvre, les moyens de calibration du classifieur comprennent des moyens de détermination d'une valeur minimale et d'une valeur maximale des niveaux d'activité échantillonnés au cours de la période d'analyse, les moyens de partitionnement opérant une partition de l'histogramme entre ces valeurs minimale et maximale. Les moyens de calibration du classifieur peuvent également comprendre des moyens de détermination d'une valeur nominale de repos et d'une valeur nominale d'effort fonction des niveaux d'activité échantillonnés au cours de la période d'analyse, les moyens de partitionnement opérant une partition de l'histogramme entre ces valeurs de repos et d'effort. La valeur nominale de repos, respectivement d'effort, peut en particulier être définie en fonction d'un pourcentage prédéterminé de la somme cumulée des niveaux échantillonnés toutes classes confondues.

On va maintenant décrire un exemple de mise en oeuvre de l'invention, en référence aux dessins annexés où les mêmes références numériques désignent d'une figure à l'autre des éléments identiques ou fonctionnellement semblables.
La Figure 1 est une vue générale de présentation du dispositif de l'invention, montrant le générateur, le myocarde et le nerf vague ainsi que les sondes utilisées.
La Figure 2 est une vue schématique par blocs correspondant aux principales fonctionnalités du générateur du dispositif de l'invention.
La Figure 3 est un histogramme, établi sur 24 heures, des valeurs moyennes d'activité du patient relevées au moyen d'un capteur accélérométrique, la figure montrant également le partitionnement de cet histogramme en quatre classes correspondant à quatre configurations de thérapie VNS différentes.
La Figure 4 représente le profil d'un histogramme, établi sur 24 heures, des valeurs moyennes d'activité du patient relevées au moyen d'un capteur physiologique de type ventilation-minute.
La Figure 5 est dérivée de l'histogramme de la Figure 4 après cumul des valeurs relevées, en particulier pour permettre la définition de niveaux d'activité de repos et d'effort.
La Figure 6 est un diagramme montrant l'évolution sur le long terme des niveaux d'activité de repos et d'effort tels que déterminés quotidiennement de la manière illustrée Figure 5.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur VNS connu.

Un tel stimulateur comprend un microprocesseur programmable pourvu de circuits pour mettre en forme et délivrer des impulsions de stimulation à des électrodes implantées. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé de l'invention est mis en oeuvre par des moyens principalement logiciels, au moyen d'algorithmes appropriés exécutés par un micro-contrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées par un même logiciel.

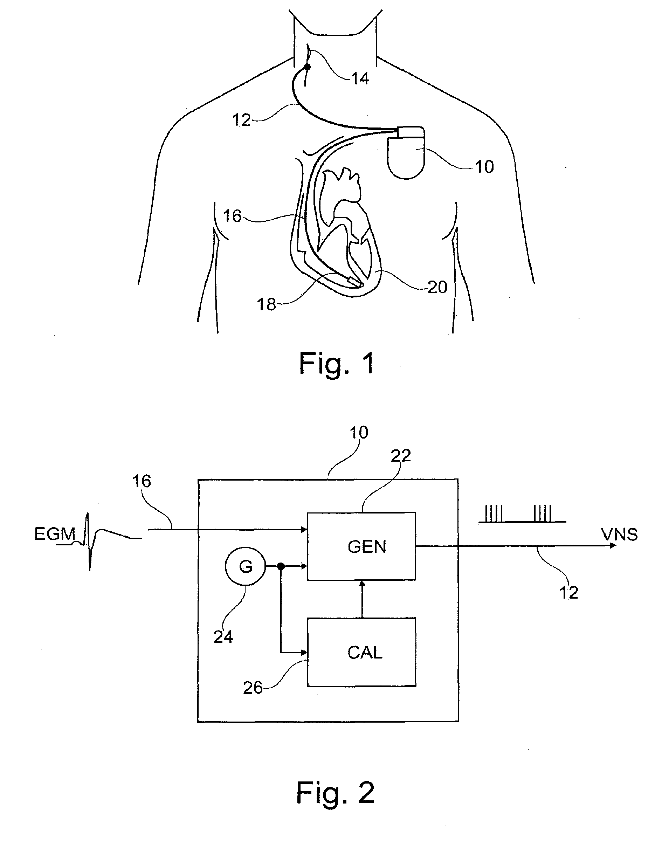

Sur la Figure 1, la référence 10 désigne le boitier d'un générateur implantable de stimulation du nerf vague. Cette stimulation est délivrée par une sonde 12 portant à sa partie distale une électrode implantée sur le nerf vague 14 et susceptible de stimuler celui-ci par application de trains d'impulsions produits par le générateur 10.

Pour permettre la délivrance d'impulsions VNS en synchronisme avec le rythme cardiaque, le générateur 10 dispose également d'une sonde cardiaque 16 pourvue à son extrémité distale d'une électrode 18 de recueil de l'activité électrique du myocarde 20, permettant de recueillir des signaux d'électrogramme endocavitaire servant ensuite à piloter le générateur 10 afin que celui-ci délivre au nerf vague 14 des impulsions de stimulation VNS au même rythme que les battements cardiaques et au moment le plus approprié de l'onde de dépolarisation cardiaque.

La Figure 2 illustre de façon schématique les principales fonctions mises en oeuvre au sein du boitier 10 dans le cadre de l'invention.

Ce boitier inclut un générateur 22 d'impulsions VNS délivrées au nerf vague via la sonde 12 en sortie. En entrée, le générateur 22 est piloté d'une part par le signal d'électrogramme EGM délivré par la sonde 16 et, d'autre part, par un signal d'activité délivré par un capteur 24.

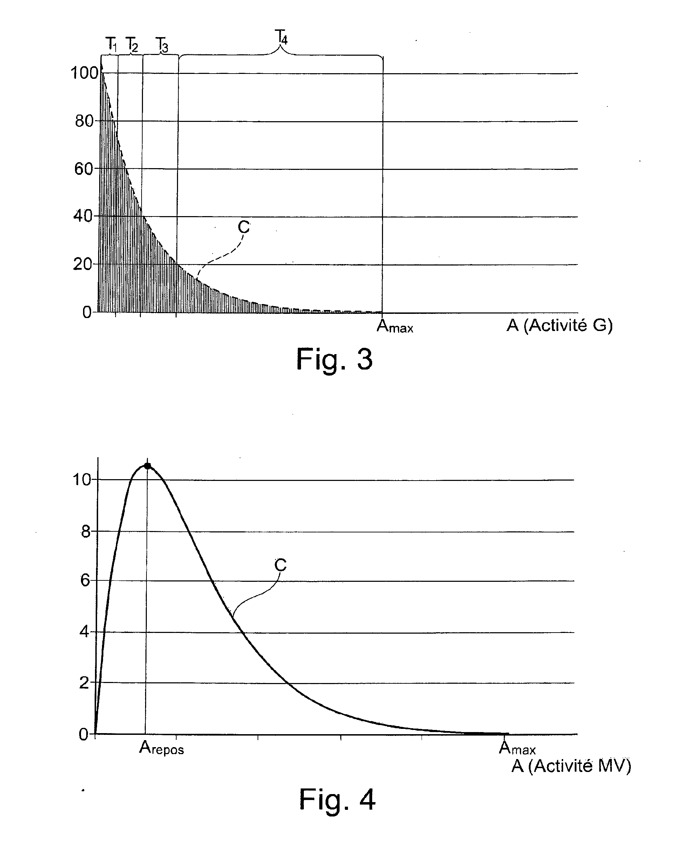

Le capteur 24 peut être, dans un premier exemple de réalisation, un capteur de mouvement du type capteur accélérométrique ou "capteur G". D'autres types de capteurs peuvent être utilisés, par exemple (comme on le décrira plus bas en relation avec les Figures 4 à 6) un capteur physiologique de type ventilation-minute ou "capteur MV", donnant une indication du niveau d'activité du patient en fonction des besoins métaboliques mesurés notamment à partir du rythme et du volume respiratoire.

Le capteur d'activité 24 est utilisé pour asservir ou moduler la thérapie VNS en fonction du niveau d'activité courant détecté, par exemple par sélection entre plusieurs niveaux d'énergie des impulsions de stimulation VNS.

Il pourra s'agir, pour certains patients, de stimuler avec une énergie décroissante au fur et à mesure que l'activité augmente, de manière à ne pas empêcher le rythme cardiaque de s'accélérer du fait de l'effort exercé par le patient.

Pour d'autres patients insuffisants cardiaques qui ont une fréquence cardiaque spontanée mal contrôlée à l'effort malgré le traitement par bêtabloquants, l'objectif thérapeutique sera, à l'inverse, de diminuer la fréquence cardiaque à l'effort, donc d'augmenter le niveau d'énergie de la thérapie VNS avec l'activité.

Dans les systèmes connus, cette modulation de la thérapie VNS résulte de la comparaison du niveau d'activité mesuré par le capteur 24 à une série de seuils successifs, ces seuils correspondant à des bornes d'une série de "classes" d'activité du patient.

De façon caractéristique de l'invention, ces différentes classes, et donc leurs bornes, ne sont plus définies de façon fixe et indifférenciée, mais de manière adaptée au patient et dynamiquement modifiable au cours du temps.

Pour cela, le dispositif comprend un circuit de calibration 26 permettant d'établir et de recalculer les seuils correspondant aux bornes des différentes classes d'activité mises en oeuvre par le générateur 22 pour sélectionner la thérapie VNS appropriée.

Ce circuit de calibration 26 opère à partir des mesures d'activité délivrées par le capteur 24 de la manière que l'on va maintenant décrire.

Des échantillons successifs de l'activité du patient sont recueillis sur une durée prédéterminée, typiquement sur les dernières 24 heures. Chaque échantillon est une moyenne du niveau d'activité mesuré par le capteur sur une durée de quelques secondes, de manière à lisser les variations instantanées du signal accélérométrique, qui peuvent être très importantes.

Le circuit de calibration 26 établit alors un histogramme de l'ensemble des valeurs ainsi mesurées et mémorisées sur 24 heures.

Un exemple de cet histogramme est illustré sur la Figure 3, avec en abscisse le niveau d'activité A correspondant à chaque valeur d'échantillon, et en ordonnée le nombre d'occurrences de chacune de ces valeurs. L'activité A varie entre un niveau nul (immobilité du patient, par exemple pendant les phases de repos ou de sommeil) et un niveau d'activité maximal Aₘₐₓ, qui peut varier de manière importante d'un jour à l'autre en fonction de l'exercice maximal produit par le patient pendant la journée en question. L'enveloppe de cet histogramme est une courbe C définissant un profil d'activité spécifique, propre (i) au patient et (ii) à la période de 24 heures considérée.

Le circuit de calibration opère ensuite une partition de cet histogramme en une pluralité de classes ou tranches, par exemple quatre tranches T₁ ... T₄, chacune correspondant à une configuration de thérapie VNS différente susceptible d'être sélectionnée par le générateur 22, notamment des thérapies différant par leur niveau d'énergie, qui décroit au fur et à mesure qu'augmente l'activité courante du patient.

La partition de l'algorithme est réalisée selon une loi prédéterminée. Par exemple, dans l'exemple illustré, les classes successives T₁ ... T₄ sont définies de manière que la somme cumulée des niveaux échantillonnés dans chaque classe soit une fraction prédéterminée, par exemple une fraction identique de 25 %, de la somme cumulée des niveaux échantillonnés toutes classes confondues : en d'autres termes, les frontières entre les classes successives T₁ ... T₄ sont choisies de manière que l'aire sous la courbe C soit la même pour chacune des classes, et égale à 25 % de l'aire totale sous la courbe C.

Les seuils définissant les frontières entre classes T₁ ... T₄ ainsi déterminés sont mémorisés par le générateur 22, qui pourra alors sélectionner telle ou telle thérapie VNS à un moment donné en fonction du niveau d'activité courant du patient à ce moment donné.

La calibration que l'on vient de décrire est avantageusement réitérée à intervalles réguliers, par exemple toutes les 24 heures ou toutes les 48 heures, de manière à intégrer une possible évolution de l'état général du patient, positive ou négative.

D'autres modes de définition dynamique des différentes classes peuvent être envisagés.

Ainsi, comme illustré sur les Figures 4 à 6, dans le cas où l'on utilise pour évaluer l'activité un capteur physiologique de type capteur de ventilation-minute MV, il est nécessaire de déterminer au préalable des valeurs d'activité au repos Aᵣₑₚₒₛ et d'activité d'effort maximal A_{effort}.

En effet, comme on peut le voir Figure 4, la courbe enveloppe C de l'histogramme obtenu à partir des mesures échantillonnées produites par le capteur MV (histogramme homologue de celui obtenu Figure 3 avec un capteur G) ne varie pas de façon monotone mais présente un pic, correspondant à l'activité au repos.

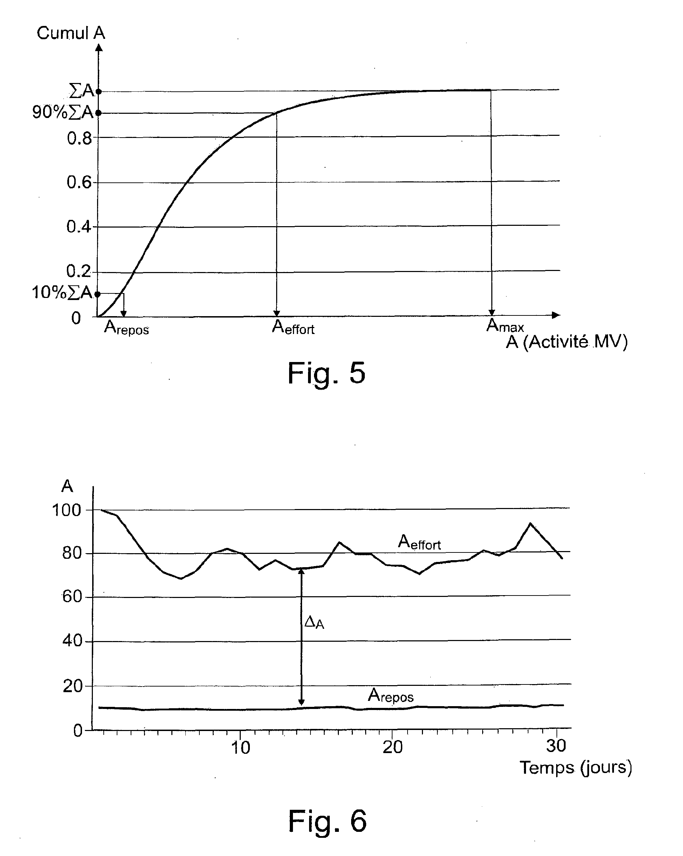

Pour déterminer la position de ce pic, le circuit de calibration calcule une somme cumulée des valeurs de l'histogramme, donnant un profil tel que celui illustré Figure 5, avec une valeur cumulée variant entre zéro et ΣA (cumul de la totalité des valeurs échantillonnées de l'histogramme).

Le niveau d'activité au repos Aᵣₑₚₒₛ est défini par exemple comme celui correspondant à 10 % de ΣA, et le niveau d'activité d'effort A_{effort} comme celui correspondant à 90 % de ΣA. Le partitionnement en classes successives est alors opéré de la manière exposée plus haut, entre les deux bornes extrêmes Aᵣₑₚₒₛ et A_{effort}.

Cette technique permet de prendre en compte la dynamique ΔA très variable d'un jour sur l'autre entre Aᵣₑₚₒₛ et A_{effort}, comme illustré Figure 6, qui représente sur une période de 30 jours l'évolution des niveaux d'activité de repos et d'effort Aᵣₑₚₒₛ et A_{effort} tels que déterminés quotidiennement de la manière exposée ci-dessus.

## Revendications

1. Un dispositif médical implantable actif pour le traitement de l'insuffisance cardiaque avec stimulation du nerf vague, VNS, comprenant :
- un générateur (10), apte à produire des salves d'impulsions VNS selon plusieurs configurations de thérapie VNS différentes sélectionnables ;
- un capteur (24), apte à générer un signal d'activité (A) fonction du niveau d'activité courante du patient ; et
- des moyens d'asservissement du générateur en fonction du signal d'activité, comprenant :
· un classifieur, apte à déterminer dans quelle classe se situe le niveau d'activité courante du patient parmi une pluralité de classes d'activité (T₁ ... T₄) définies par des bornes respectives du niveau d'activité ; et
· un contrôleur, apte à sélectionner une configuration de thérapie VNS respective en fonction de la classe d'activité déterminée par le classifieur,
dispositif **caractérisé en ce que** les bornes définissant les classes d'activité sont des bornes dynamiquement modifiables, et **en ce qu'**il comprend en outre :
- des moyens (26) de calibration du classifieur, comprenant :
· des moyens d'analyse historique des niveaux d'activité courants successifs du patient pris au cours d'une période d'analyse prédéterminée ; et
· des moyens de définition des bornes des classes d'activité (T₁ ... T₄) en fonction des résultats de ladite analyse historique.

2. Le dispositif de la revendication 1, comprenant en outre des moyens de mise à jour du classifieur, aptes à activer de manière réitérée les moyens de calibration du classifieur.

3. Le dispositif de la revendication 2, dans lequel les moyens de mise à jour comprennent des moyens aptes à réactiver à intervalles réguliers les moyens de calibration du classifieur.

4. Le dispositif de la revendication 1, dans lequel les moyens de calibration du classifieur comprennent :
· des moyens d'échantillonnage des niveaux d'activité courants successifs du patient au cours de ladite période d'analyse ;
· des moyens de construction d'un histogramme (C) des niveaux d'activité échantillonnés ; et
· des moyens de partitionnement de l'histogramme en ladite pluralité de classes d'activité (T₁ ... T₄).

5. Le dispositif de la revendication 4, dans lequel les moyens d'échantillonnage des niveaux d'activité courants échantillonnent un niveau moyen d'activité au cours de chaque période d'échantillonnage respective.

6. Le dispositif de la revendication 4, dans lequel les moyens de partitionnement de l'histogramme comprennent des moyens aptes à définir les bornes entre classes successives de manière que la somme cumulée des niveaux échantillonnés dans chaque classe soit une fraction prédéterminée de la somme cumulée des niveaux échantillonnés toutes classes confondues.

7. Le dispositif de la revendication 6, dans lequel ladite fraction prédéterminée est une fraction identique pour toutes les classes.

8. Le dispositif de la revendication 4, dans lequel les moyens de calibration du classifieur comprennent en outre :
· des moyens de détermination d'une valeur minimale (Aₘᵢₙ) et d'une valeur maximale (Aₘₐₓ) desdits niveaux d'activité échantillonnés au cours de la période d'analyse,
et en ce que les moyens de partitionnement opèrent une partition de l'histogramme entre lesdites valeurs minimale et maximale.

9. Le dispositif de la revendication 4, dans lequel les moyens de calibration du classifieur comprennent en outre :
· des moyens de détermination d'une valeur nominale de repos (Aᵣₑₚₒₛ) et d'une valeur nominale d'effort (A_{effort}) fonction desdits niveaux d'activité échantillonnés au cours de la période d'analyse,
et en ce que les moyens de partitionnement opèrent une partition de l'histogramme entre lesdites valeurs de repos et d'effort.

10. Le dispositif de la revendication 9, dans lequel la valeur nominale de repos, respectivement d'effort, est définie en fonction d'un pourcentage prédéterminé de la somme cumulée des niveaux échantillonnés toutes classes confondues.

## Patentansprüche

1. Aktive implantierbare medizinische Vorrichtung zur Behandlung von Herzinsuffizienz mit Vagusnervstimulation VNS, umfassend:
- einen Generator (10), der geeignet ist, VNS-Impulssalven gemäß mehreren, verschiedenen, wählbaren VNS-Therapie-Konfigurationen zu erzeugen;
- einen Sensor (24), der geeignet ist, ein Aktivitätssignal (A) zu erzeugen, das vom üblichen Aktivitätsniveau des Patienten abhängig ist; und
- Mittel zum Steuern des Generators in Abhängigkeit vom Aktivitätssignal, umfassend:
• einen Klassifizierer, der geeignet ist, aus einer Vielzahl von Aktivitätsklassen (T₁ ... T₄), die durch die jeweiligen Grenzwerte des Aktivitätsniveaus definiert sind, zu bestimmen, in welcher Klasse sich das übliche Aktivitätsniveau des Patienten befindet; und
• einen Regler, der geeignet ist, in Abhängigkeit von der von dem Klassifizierer bestimmten Aktivitätsklasse, jeweils eine VNS-Therapie-Konfiguration auszuwählen,
wobei die Vorrichtung **dadurch gekennzeichnet ist, dass** es sich bei den Grenzwerten, die die Aktivitätsklassen definieren, um dynamisch modifizierbare Grenzwerte handelt, und dadurch, dass sie ferner umfasst:
- Mittel (26) zum Kalibrieren des Klassifizierers, umfassend:
• Mittel zur Verlaufsanalyse der aufeinander folgenden üblichen Aktivitätsniveaus des Patienten, die im Verlauf eines vorbestimmten Analysezeitraums aufgezeichnet wurden; und
• Mittel zur Definition der Grenzwerte der Aktivitätsklassen (T₁ ... T₄) in Abhängigkeit von den Ergebnissen der Verlaufsanalyse.

2. Vorrichtung nach Anspruch 1, ferner umfassend Mittel zur Aktualisierung des Klassifizierers, die geeignet sind, die Mittel zum Kalibrieren des Klassifizierers wiederholt zu aktivieren.

3. Vorrichtung nach Anspruch 2, wobei die Mittel zur Aktualisierung Mittel umfassen, die geeignet sind, die Mittel zum Kalibrieren des Klassifizierers in regelmäßigen Abständen zu reaktivieren.

4. Vorrichtung nach Anspruch 1, wobei die Mittel zum Kalibrieren des Klassifizierers umfassen:
• Mittel zum Erfassen der aufeinander folgenden üblichen Aktivitätsniveaus des Patienten im Verlauf des Analysezeitraums;
• Mittel zum Erstellen eines Histogramms (C) der erfassten Aktivitätsniveaus; und
• Mittel zum Aufteilen des Histogramms in die Vielzahl von Aktivitätsklassen (T₁ ... T₄).

5. Vorrichtung nach Anspruch 4, wobei die Mittel zum Erfassen der üblichen Aktivitätsniveaus im Verlauf jedes Erfassungszeitraums jeweils ein mittleres Aktivitätsniveau erfassen.

6. Vorrichtung nach Anspruch 4, wobei die Mittel zum Aufteilen des Histogramms Mittel umfassen, die geeignet sind, die Grenzwerte zwischen aufeinander folgenden Klassen derart zu definieren, dass die Gesamtsumme der erfassten Niveaus in jeder Klasse ein vorbestimmter Bruchteil der Gesamtsumme der erfassten Niveaus aller Klassen ist.

7. Vorrichtung nach Anspruch 6, wobei der vorbestimmte Bruchteil ein Bruchteil ist, der für alle Klassen identisch ist.

8. Vorrichtung nach Anspruch 4, wobei die Mittel zum Kalibrieren des Klassifizierers ferner umfassen:
• Mittel zum Bestimmen eines Minimalwerts (A_{Min}) und eines Maximalwerts (A_{Max}) der Aktivitätsniveaus, die im Verlauf des Analysezeitraums erfasst wurden,
und dadurch, dass die Mittel zum Aufteilen eine Aufteilung des Histogramms zwischen den Minimal- und Maximalwerten durchführen.

9. Vorrichtung nach Anspruch 4, wobei die Mittel zum Kalibrieren des Klassifizierers ferner umfassen:
• Mittel zum Bestimmen eines Ruhe-Nominalwerts (A_{Ruhe}) und eines Anstrengungs-Nominalwerts (A_{Anstrengung}) in Abhängigkeit von den Aktivitätsniveaus, die im Verlauf des Analysezeitraums erfasst wurden,
und dadurch, dass die Mittel zum Aufteilen eine Aufteilung des Histogramms zwischen den Ruhe- und Anstrengungs-Nominalwerten durchführen.

10. Vorrichtung nach Anspruch 9, wobei der Ruhe- bzw. Anstrengungs-Nominalwert in Abhängigkeit von einem vorbestimmten Prozentsatz der Gesamtsumme der erfassten Niveaus aller Klassen definiert wird.

## Claims

1. An active implantable medical device for treating heart failure with vagus nerve stimulation, VNS, comprising:
- a generator (10), adapted to produce VNS pulse bursts according to several different selectable VNS therapy configurations;
- a sensor (24), adapted to generate a signal of activity (A) that is function of the level of current activity of the patient; and
- means for controlling the generator as a function of the signal of activity, comprising:
. a classifier, adapted to determine in which class is located the level of current activity of the patient among a plurality of classes of activity (T₁...T₄) defined by respective boundaries of the level of activity; and
. a controller, adapted to select a respective VNS therapy configuration as a function of the class of activity determined by the classifier,
the device being **characterized in that** the boundaries defining the classes of activity are dynamically modifiable boundaries, and **in that** it further includes:
- means (26) for calibrating the classifier, comprising:
. means for historical analysis of the successive current levels of activity of the patient taken during a predetermined period of analysis; and
. means for defining the boundaries of the classes of activity (T₁...T₄) as a function of the results of said historical analysis.

2. The device of claim 1, further comprising means for updating the classifier, adapted to activate the classifier calibration means in a reiterated manner.

3. The device of claim 2, wherein the updating means comprise means adapted to reactivate the classifier calibration means at regular intervals.

4. The device of claim 1, wherein the classifier calibration means comprise:
. means for sampling the successive current levels of activity of the patient during said period of analysis;
. means for constructing an histogram (C) of the sampled levels of activity ; and
. means for partitioning the histogram into said plurality of classes of activity (T₁...T₄).

5. The device of claim 4, wherein the means for sampling the current levels of activity sample a mean level of activity during each respective period of sampling.

6. The device of claim 4, wherein the histogram partitioning means comprise means adapted to define the boundaries between successive classes so that the cumulated sum of the sampled levels in each class is a predetermined fraction of the cumulated sum of the sampled levels, all classes taken together.

7. The device of claim 6, wherein said predetermined fraction is an identical fraction for all the classes.

8. The device of claim 4, wherein the classifier calibration means further comprise:
. means for determining a minimum value (Aₘᵢₙ) and a maximum value (Aₘₐₓ) of said sampled levels of activity during the period of analysis,
and in that the partitioning means operate a partition of the histogram between said minimum and maximum values.

9. The device of claim 4, wherein the classifier calibration means further comprise:
. means for determining a nominal rest value (Aᵣₑₛₜ) and a nominal effort value (A_{effort}) that are function of said levels of activity sampled during the period of analysis,
and in that the partitioning means operate a partition of the histogram between said rest and effort values.

10. The device of claim 9, wherein the nominal rest, respectively effort, value is defined as a function of a predetermined percentage of the cumulated sum of the sampled levels, all classes taken together.
